# EUROPEAN PATENT SPECIFICATION

(11) **EP 2 417 907 B1**
(45) Date of publication and mention of the grant of the patent: **31.10.2012**
(21) Application number: 10425277.0
(22) Date of filing: 12.08.2010
(51) Int. Cl.: A61B 5/103, A61B 5/107, A61B 9/00, A61B 19/00

(54) **Multi-functional instrument for medical use, particularly for use in orthopedy**
Multifunktionales Instrument für medizinische Zwecke, insbesondere für den Einsatz in der Orthopädie
Instrument multi-fonctionnels à usage médical, en particulier pour une utilisation en orthopédie

(43) Date of publication of application: 15.02.2012
(73) Proprietor: Amenduni Gresele, Massimo, 36100 Vicenza (IT)
(72) Inventor: Zona, Mauro, 10153 Torino (IT); Giglio, Gennaro, 80030 Camposano (Napoli) (IT)
(74) Representative: Gallarotti, Franco

(56) References cited:
- GB-A- 2 221 535
- US-A- 5 119 521
- US-A1- 2004 165 375
- US-A1- 2009 038 168

## Description

The present invention refers to a multifunctional instrument for medical use, particularly for use in orthopedy, comprising a handle having an extended body ending with a head which may be used as a reflex hammer head, wherein said extended body is divided longitudinally into two arms which are mutually articulated around a central axis of said head so as to be useable as goniometer arms, also wherein the head incorporates a rolled tape measure, and wherein at least one stimulation tip is accommodated in one of said arms.

A multifunctional instrument having the abovementioned characteristics is described in document GB-A-2 221 535. Such known device doubtlessly has the advantage of providing the physician with a plurality of instruments and tools incorporated in a single easily portable instrument. However, the prior art solution is not entirely satisfactory in terms of efficiency and comfort in use thereof.

Thus, the object of the present invention is that of improving the prior art device, by providing a multifunctional instrument which is particularly efficient in use, comfortable to use and having a simple construction structure and being relatively inexpensive.

With the aim of attaining the abovementioned objective, the invention, which is defined in claim 1 and its dependent claims, has the object of a multifunctional medical instrument having all the characteristics indicated above and further characterized in that:
- the abovementioned head has a generally T-shaped configuration, with a central part and two opposite tapered ends, projecting from said central part,
- said T-shaped head is in a single piece with a first of said arms,
- the two abovementioned arms have flat mutual contact surfaces which are parallel to the axis of mutual articulation of the arms and they are provided with magnetic effect coupling elements, for holding the arms in the condition of contact thereof,
- the abovementioned tape measure is accommodated wound in a cavity of the head and it is also guided in a longitudinal slot of said first arm, so that the free end of the tape projects from the free end of said first arm, to allow seizure thereof, such tape substantially having a barycentric position with respect to the instrument, hence said tape may also be used as a plummet by seizing it by its free end and using the instrument as a lower mass, and
- said head is traversed from one face to the other by an opening substantially centred on the axis of mutual articulation of said arms.

Such central opening allows laying the head of the instrument for example against the surface of a radiographic film or a radiographic film viewer by centring the axis of the goniometer on a desired point of the film.

Preferably, the second arm has - on the flat surface thereof for contact with the first arm - an accommodation for one from among a stimulation brush and said stimulation tip, while the other from among said brush and said tip is slidably mounted in a longitudinal slot of the second arm, and it is moveable by means of a slidable button between a retracted position and a position projecting from the free end of the second arm.

Still preferably, the abovementioned slidable actuation button is mounted on the second arm on the flat surface for contact with the first arm and it has an edge which however projects outside the two arms even when they are in the condition of contact thereof.

Still preferably, at least one of the two arms has - on an external face thereof - a linear graduated scale for measuring lengths. In the preferred embodiment, both arms have a graduated scale, which may for example be a millimetre scale on one arm and an inch scale on the other arm.

Still in the case of the preferred embodiment, the tape measure is not provided with a return spring and it is provided with a rewinding crown having an edge projecting outside the head of the instrument.

Due to the characteristics indicated above, the instrument according to the invention has several advantages. First and foremost, the head of the instrument has an ideal configuration for use as a reflex hammer, with two opposite tapered ends which are preferably covered with a coating made of deformable material, such as rubber or the like. The abovementioned head is made in a single piece integral with the first of the two arms, to the advantage of simplicity and low manufacturing costs. Should the body of the instrument be made of metal material, the head with the respective arm may for example be obtained through casting. The division of the extended handle of the instrument into two separate arms is obtained through a plane parallel to the articulation axis of the arms (and not orthogonal to such plane, like in the case of the prior art solution) hence allowing using the two flat faces for mutual contact of the two arms for bearing magnetic effect coupling elements and for obtaining the accommodations for several components, as described in detail hereinafter. Furthermore, contrary to the prior art device, where the tape measure projects from an opening provided on the head of the instrument, in the case of the present invention the tape is guided through a longitudinal slot formed along one of the two arms and projects from the free end of such arm and it may also be used as plummet. The use of the stimulation tip is also particularly easy and comfortable, due to the prearrangement of a slidable actuation button which allows projecting the tip from an end of one of the two arms of the instrument. Also as indicated, one of the arms is also used for obtaining an accommodation for a stimulation brush, which allows further completing the kit available for the physician. In order to guarantee maximum reliability and duration of the instrument, the device according to the invention does not use a return spring for the tape measure in the wound condition, given that springs of this type are subjected to the risk of malfunctioning or failures, but it is provided with a winding crown which is however easy and comfortable to use for the user and eliminates any problem of reliability of the aforedescribed type right from the source.

Further characteristics and advantages of the invention shall be clear from the description that follows with reference to the attached drawings, purely provided by way of non-limiting example, wherein:
- figures 1, 2 are two perspective views - from opposite sides - of a preferred embodiment of the instrument according to the invention, with the arms in the contact condition,
- figure 3 is a perspective view of the instrument of figures 1, 2, with the arms in a diverged condition, and
- figure 4 is an exploded perspective view of the instrument according to the invention.

With reference to the drawings, number 1 - in its entirety - indicates a multifunctional instrument for medical use, useable particularly by orthopedic physicians, comprising a handle, indicated in its entirety with reference number 2, which has an extended body ending with an enlarged T-shaped head 3 having a central part 4 and two opposite and tapered ends 5, each of which is provided with a cap-like cover, for example made of rubber.

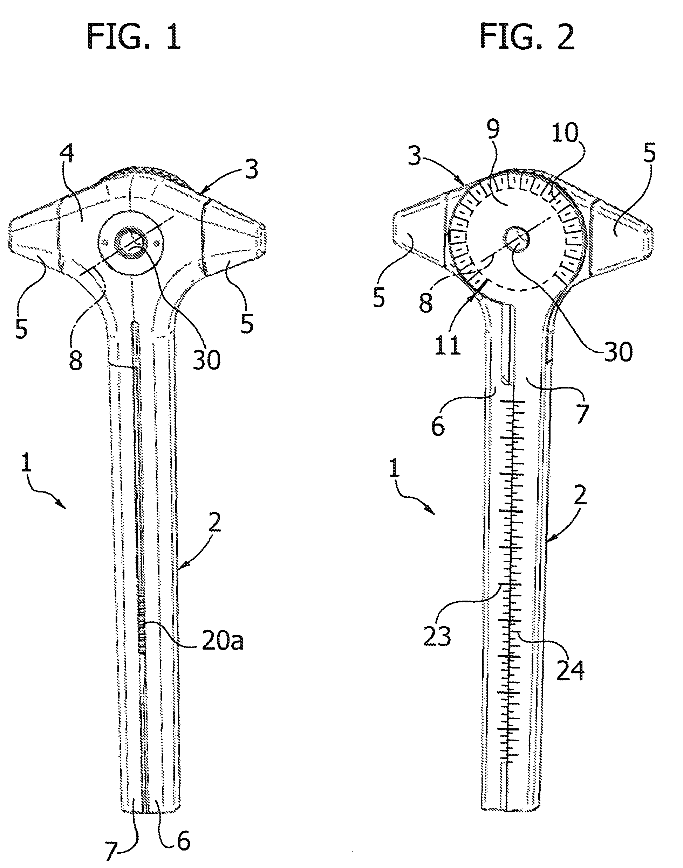

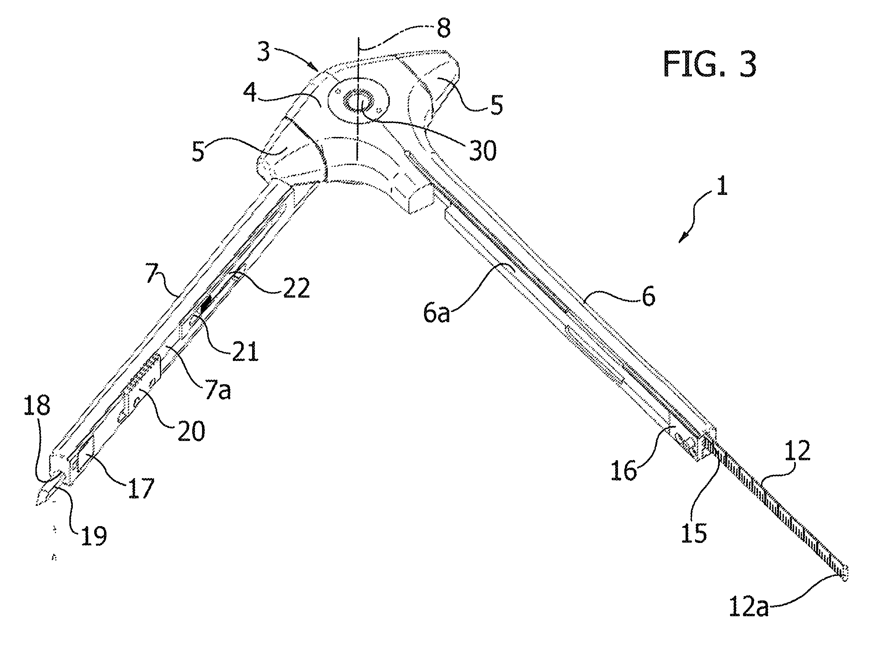

The extended body 2 is divided longitudinally into two arms 6, 7 which are mutually articulated around a central axis 8 of the head 3, so as to be moveable between the contact condition shown in figures 1, 2 and the diverged condition shown in figure 3.

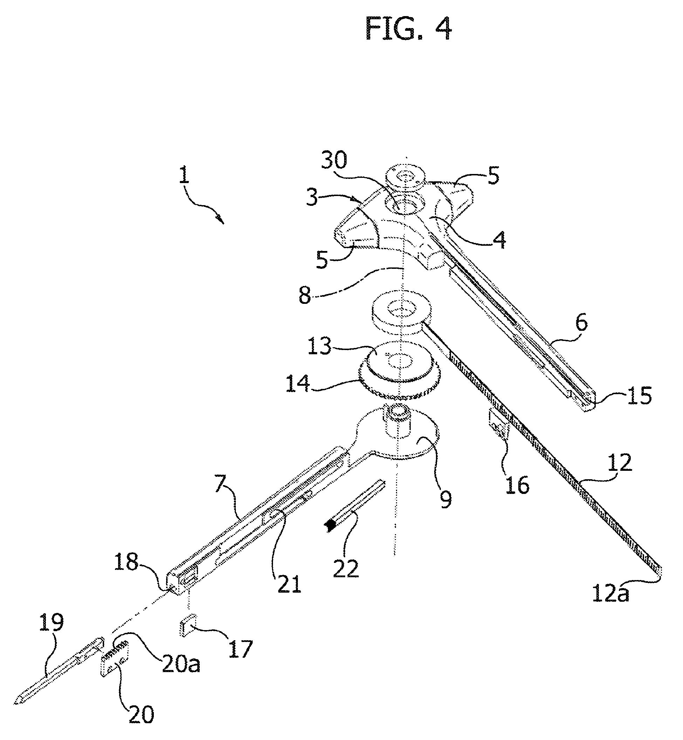

As clearly illustrated in figure 3 and also in figure 4, the body of the head 3 is made in a single piece with the first arm 6, while the second arm 7 also has a head, but shaped to form a flat disc 9 (figure 4) which is juxtaposed and articulated around the axis 8 to the head 3. In its entirety, the head of the instrument is traversed from one face to the other by an opening substantially centred on the axis of mutual articulation of said arms. Such opening allows laying the head of the instrument for example against the surface of a radiographic film or a radiographic film viewer by centring the axis of the goniometer on a desired point of the film.

The possibility of moving the arms 6, 7 between the contact condition illustrated in figures 1, 2 and the diverged condition illustrated in figure 3 allows using such arms as goniometer arms, for measuring angles, in cooperation with a circumferential graduated scale 10 prearranged on the outer face of the disc 9 and with an index 11 prearranged on the arm 6. In the specific illustrated example, the graduated scale is extended over an angular range equivalent to 270°.

All the abovementioned components are for example made of metal material or synthetic material.

As observable in figure 4, the instrument incorporates a tape measure 12 which is wound on a drum 13 received rotatably in a cavity obtained in the face of the head 3, not shown in figure 4, which is covered by the disc 9 borne by the second arm 7. The drum 13 comprises a crown with a knurled edge 14 which projects outside the head 3 (see figure 1, 2) with the aim of allowing controlling a rewinding of the tape after the latter has been extracted. Furthermore, as observable in figures 3, 4, the tape measure 12 is guided into a longitudinal slot 15 of the first arm 6 and projects from the free end of the arm 6. The tape measure 12 may thus be extracted by seizing an end 12a which is slightly enlarged (so as not to retract into the slot 15). Such tape, according to the invention, is also used as a plummet, by holding it by the end 12a and using the instrument with the arms closed as a lower mass (the tape 12 having a barycentric position with respect to the instrument).

As clearly observable in figure 3, the plane for dividing the extended body 2 into the two arms 6, 7 is a plane parallel to the articulation axis 8 of such arms. Therefore, the two arms 6, 7 have flat contact surfaces 6a, 7a parallel to the axis 8. The two arms 6, 7 are provided - at the free ends thereof, on the respective faces 6a, 7a, with two mutual magnetic effect coupling elements 16, 17, for holding the two arms 6, 7 in the closed condition. Starting from such condition, the two arms may be diverged by applying a slight force, enough to overcome the magnetic attraction force between the elements 16, 17.

The second arm 7 has a longitudinal slot 18 in which a stimulation tip 19 - which may be moved between a retracted position in the arm 7 and an extracted position (shown in figure 3) through an actuation button 20 - is mounted slidably. As observable in figure 4, the button 20 is constituted by a flat plate fixed, for example by means of screws, to the interior end of the tip 19 and juxtaposed to the flat surface 7a of the arm 7. The plate 20 has a knurled edge 20a projecting outside the two arms 6, 7, when the latter are at contact, so as to be however accessible even at the contact condition of the two arms 6, 7 (figure 1).

Still referring to the figures figure 3, 4, the flat surface 7a of the second arm 7 has an accommodation 21 for a stimulation brush 22.

Furthermore, referring to figure 2, the two arms 6, 7 have - on a face thereof - linear graduated scales 23, 24, for example in millimetres and in inches.

As clearly observable from the description above, the instrument according to the invention has several innovative characteristics which make it ideal both in terms of efficiency and comfort in use. Furthermore, the structure of the instrument according to the invention is configured so as to be easy to construct and be relatively inexpensive.

Naturally, without prejudice to the principle of the invention, the construction details and the embodiments may widely vary with respect to what has been described and illustrated purely by way of example, without departing from the scope of protection of the present invention as defined by the attached claims.

## Claims

1. Multifunctional medical instrument, particularly for use in orthopedy, comprising a handle (2) having an extended body ending with a head (3) useable as a reflex hammer head,
- wherein said extended body (2) is divided longitudinally into two arms (6, 7) which are mutually articulated around a central axis (8) of said head (3), so as to be useable as goniometer arms,
- wherein said head (3) incorporates a rolled tape measure (12), and
- wherein at least one stimulation tip (19) is accommodated in one of said arms (7),
said instrument further being **characterised in that**:
- the abovementioned head (3) has a generally T-shaped configuration, with a central part (4) and two opposite tapered ends (5), projecting from said central part (4),
- said T-shaped head (3) is made in a single piece with a first (6) of said arms (6, 7),
- the two abovementioned arms (6, 7) have flat mutual contact surfaces (6a, 7a) which are parallel to the axis (8) of mutual articulation of the arms (6, 7) and they are provided with magnetic effect coupling elements (16, 17), for holding the arms (6, 7) in the condition of contact thereof,
- the abovementioned tape measure (12) is accommodated wound in a cavity of the head (3) and it is also guided in a longitudinal slot (15) of said first arm (6), so that the free end of the tape (12) projects from the free end of said first arm (6), such tape substantially having a barycentric position with respect to the instrument, hence said tape (12) may also be used as plummet by holding it by its free end and using the instrument as a lower mass, and
- said head has a flat face which may be laid on a flat surface and it is traversed from one face to the other by a central opening substantially centred on the axis of mutual articulation of said arms.

2. Instrument according to claim 1, **characterised in that** the second arm (7) has - on the flat surface (7a) thereof for contact with the first arm (6) - an accommodation (21) for one from among a stimulation brush (22) and said stimulation tip (19) and **in that** the other from among said brush (22) and said tip (19) is slidably mounted in a longitudinal slot (18) of the second arm (7), and it is moveable - by means of a slidable button (17) between a retracted position and a position projecting from the free end of the second arm (7).

3. Instrument according to claim 1, **characterised in that** the abovementioned slidable button (20) is mounted on the second arm (7) on the flat surface (7a) for contact with the first arm (6) and it has an engagement edge (20a) which however projects outside the two arms (1, 2) even when they are in the condition of contact thereof.

4. Instrument according to claim 1, **characterised in that** at least one of the two arms (6, 7) has - on an external face thereof - a linear graduated scale (23,24) for measuring lengths.

5. Instrument according to claim 1, **characterised in that** the tape measure (12) is not provided with a return spring and it is provided with a rewinding crown (14) having an edge projecting outside the head (3).

6. Instrument according to claim 1, **characterised in that** the opposite ends (5) of the head (3) are provided with coatings made of deformable material.

7. Instrument according to claim 1, **characterised in that** the second arm (7) has an end articulated to the first arm (6) in form of a flat disc (9), and that such disc (9) covers a cavity made in the head (3) for accommodating the wound tape measure (12) and bears - on the outer face thereof - a circumferential graduated scale cooperating with an index borne by said first arm (6), for the use of the instrument as a goniometer.

## Patentansprüche

1. Multifunktionales medizinisches Instrument, insbesondere für die Verwendung in der Orthopädie, umfassend einen Griff (2), der ein erweitertes Körperende mit einem Kopf (3) hat, der als Reflexhammerkopf verwendbar ist,
- wobei der erweiterte Körper (2) in Längsrichtung in zwei Arme (6, 7) unterteilt ist, die gegeneinander um eine zentrale Achse (8) des Kopfes (3) geschwenkt werden können, um so als Goniometerarme verwendbar zu sein,
- wobei der Kopf (3) ein Rollenmaßband (12) enthält, und
- wenigstens eine Stimulationsspitze (19) in einem der Arme aufgenommen ist,
- wobei das Instrument weiterhin **dadurch gekennzeichnet ist, dass**:
- der oben erwähnte Kopf (3) eine im wesentlichen T-förmige Gestalt mit einem zentralen Teil (4) und zwei gegenüberliegende sich verjüngenden Enden (5) hat, die von dem zentralen Teil (4) hervorragen,
- wobei der T-förmige Kopf (3) aus einem einzigen Stück mit einem ersten (6) der Arme (6, 7) ausgebildet ist,
- die beiden oben erwähnten Arme (6, 7) gegenseitig flache Kontaktflächen (6a, 7a) haben, die parallel zu der Achse (8) für das gegenseitige Schwenken der Arme (6, 7) sind und mit Magneteffekt-Kopplungselementen (16, 17) zum Halten der Arme (6, 7) in einem Zustand ihrer Berührung versehen sind,
- das oben erwähnte Maßband (12) aufgerollt in einem Hohlraum des Kopfes (3) aufgenommen ist und zudem in einem Längsschlitz (15) des ersten Armes (6) derart geführt ist, dass das freie Ende des Bandes (12) von dem freien Ende des ersten Armes (6) hervorragt, wobei das Band im wesentlichen eine baryzentrische Position im Bezug auf das Instrument hat, womit dieses Band (12) auch als Lot verwendet werden kann, indem es an seinem freien Ende gehalten und das Instrument als untere Masse verwendet wird, und
- der Kopf eine flache Fläche hat, die auf eine flache Oberfläche gelegt werden kann, und den von der einen zur anderen Seite eine zentrale Öffnung durchläuft, die im wesentlichen auf der Achse für das gegenseitige Schwenken der Arme zentriert ist.

2. Instrument nach Anspruch 1, **dadurch gekennzeichnet, dass** der zweite Arm (7) - auf seiner flachen Oberfläche (7a) für den Kontakt mit dem ersten Arm (6) - eine Aufnahme (21) entweder für einen Stimulationspinsel (22) oder die Stimulationsspitze (19) hat und das entsprechende andere Element von Pinsel (22) und Spitze (19) verschiebbar in einem Längsschlitz (18) des zweiten Arms (7) aufgenommen und mit Hilfe eines verschiebbaren Knopfes (17) zwischen einer zurückgezogenen Stellung und einer Stellung verschiebbar ist, die von dem freien Ende des zweiten Arms (7) hervorragt.

3. Instrument nach Anspruch 1, **dadurch gekennzeichnet, dass** der oben erwähnte verschiebbare Knopf (20) an dem zweiten Arm (7) auf der flachen Oberfläche (7a) für den Kontakt mit dem ersten Arm (6) angebracht ist und einen Eingriffsrand (20a) hat, der jedoch aus den beiden Armen (1, 2) auch dann hervorragt, wenn sie sich einander berühren.

4. Instrument nach Anspruch 1, **dadurch gekennzeichnet**, das wenigstens einer der beiden Arme (6, 7) - auf seiner Außenfläche - eine lineare Skala (23, 24) zur Längenmessung hat.

5. Instrument nach Anspruch 1, **dadurch gekennzeichnet, dass** das Maßband (12) nicht mit einer Rückholfeder versehen ist und mit einer Aufrollkrone (14) ausgestattet ist, die einen Rand aufweist, der aus dem Kopf (3) hervorragt.

6. Instrument nach Anspruch 1, **dadurch gekennzeichnet, dass** die gegenüberliegenden Enden (5) des Kopfes (3) mit Beschichtungen eines verformbaren Materials versehen sind.

7. Instrument nach Anspruch 1, **dadurch gekennzeichnet, dass** der zweite Arm (7) ein Ende aufweist, das mit dem ersten Arm (6) gelenkig verbunden ist und die Gestalt einer flachen Scheibe (9) hat, und diese Scheibe (9) einen Hohlraum verschließt, der in dem Kopf (3) ausgebildet ist, um das aufgewickelte Maßband (12) aufzunehmen und - an seiner Außenfläche - eine in Umfangsrichtung verlaufende Maßskala trägt, die mit einem Index zusammenwirkt, der von dem ersten Arm (6) gehalten ist, um das Instrument als Goniometer zu verwenden.

## Revendications

1. Instrument médical multifonctionnel, en particulier destiné à être utilisé en orthopédie, comprenant un manche (2) ayant un corps alongé se terminant par une tête (3) pouvant être utilisée en tant que tête de marteau à réflexes,
dans lequel ledit corps étendu (2) est divisé longitudinalement en deux bras (6, 7) qui sont mutuellement articulés autour d'un axe central (8) de ladite tête (3), afin d'être utilisés en tant que bras de goniomètre,
dans lequel ladite tête (3) comprend un ruban métrique enroulé (12), et dans lequel au moins une pointe de stimulation (19) est logée dans l'un desdits bras (7),
ledit instrument étant en outre **caractérisé en ce que** :
la tête (3) mentionnée ci-dessus a une configuration généralement en forme de T, avec une partie centrale (4) et deux extrémités comiques rétrécies opposées (5), faisant saillie de ladite partie centrale (4),
ladite tête en forme de T (3) est réalisée d'un seul tenant avec un premier (6) desdits bras (6, 7),
les deux bras (6, 7) mentionnés ci-dessus ont des surfaces de contact mutuel plates (6a, 7a) qui sont parallèles à l'axe (8) d'articulation mutuelle des bras (6, 7) et sont munies d'éléments de couplage à effet magnétique (16, 17) pour maintenir les bras (6, 7) dans la condition de leur contact,
le ruban métrique (12) mentionné ci-dessus est logé en étant enroulé dans une cavité de la tête (3) et est également guidé dans une fente longitudinale (15) dudit premier bras (6), de sorte que l'extrémité libre du ruban (12) fait saillie à partir de l'extrémité libre dudit premier bras (6), un tel ruban ayant sensiblement une position barycentrique par rapport à l'instrument, par conséquent, ledit ruban (12) peut également être utilisé en tant que fil à plomb en le tenant par son extrémité libre et utiliser l'instrument en tant que masse inférieure, et
ladite tête a une face plate qui peut être posée sur une surface plate et est traversée d'une face à l'autre par une ouverture centrale sensiblement centrée sur l'axe d'articulation mutuelle desdits bras.

2. Instrument selon la revendication 1, **caractérisé en ce que** le second bras (7) a - sur sa surface plate (7a) pour le contact avec le premier bras (6) - un logement (21) pour l'une parmi une brosse de stimulation (22) et ladite pointe de stimulation (19) et **en ce que** l'autre parmi ladite brosse (22) et ladite pointe (19) est montée de manière coulissante dans une fente longitudinale (18) du second bras (7), et est mobile - au moyen d'un bouton coulissant (17) entre une position rétractée et une position faisant saillie de l'extrémité libre du second bras (7).

3. Instrument selon la revendication 1, **caractérisé en ce que** le bouton coulissant (20) mentionné ci-dessus est monté sur le second bras (7) sur la surface plate (7a) pour le contact avec le premier bras (6) et il a un bord de mise en prise (20a) qui fait cependant saillie vers l'extérieur des deux bras (1, 2) même lorsqu'ils sont dans leur condition de contact.

4. Instrument selon la revendication 1, **caractérisé en ce qu'**au moins l'un des deux bras (6, 7) a - sur sa face externe - une échelle graduée linéaire (23, 24) pour mesurer des longueurs.

5. Instrument selon la revendication 1, **caractérisé en ce que** le ruban métrique (12) n'est pas prévu avec un ressort de rappel et il est doté d'une couronne de rembobinage (14) ayant un bord faisant saillie à l'extérieur de la tête (3).

6. Instrument selon la revendication 1, **caractérisé en ce que** les extrémités opposées (5) de la tête (3) sont équipées de revêtements réalisés à partir d'un matériau déformable.

7. Instrument selon la revendication 1, **caractérisé en ce que** le second bras (7) a une extrémité articulée par rapport au premier bras (6) se présentant sous la forme d'un disque plat (9), et **en ce qu'**un tel disque (9) recouvre une cavité réalisée dans la tête (3) pour loger le ruban métrique (12) enroulé et supporte - sur sa face externe - une échelle graduée circonférentielle coopérant avec un indice supporté par ledit premier bras (6), pour utiliser l'instrument en tant que goniomètre.
